# EUROPEAN PATENT SPECIFICATION

(11) **EP 1 885 364 B1**
(45) Date of publication and mention of the grant of the patent: **06.01.2010**
(21) Application number: 06763131.7
(22) Date of filing: 12.05.2006
(51) Int. Cl.: A61K 31/4745, C07D 519/00, C07K 5/00, A61P 35/00

(54) **HALOGENATED QUINDOLINE DIMERS FOR THE TREATMENT OF CANCER**
HALOGENIERTE CHINDOLIN-DIMERE ZUR BEHANDLUNG VON KREBS
DIMERES DE QUINDOLINE HALOGNES POUR LE TRAITEMENT DU CANCER

(30) Priority: 20.05.2005 DE 102005023978
(43) Date of publication of application: 13.02.2008
(73) Proprietor: Crystax Pharmaceuticals, S.L., 08028 Barcelona (ES)
(72) Inventor: AYMAMÍ BOFARULL, Juan, E-08790 Gelida (barcelona) (ES); NAVARRO MUÑOZ, Isabel, E-08917 Badalona (barcelona) (ES)
(74) Representative: Barlocci, Anna
(86) International application number: PCT/EP2006/062277
(87) International publication number: WO 2006/122910

(56) References cited:
- WO-A-98/45272
- WO-A-2005/054236
- YANG S-W ET AL: "Synthesis and Biological Evaluation of Analogues of Cryptolepine, an Alkaloid Isolated from the Suriname Rainforest" JOURNAL OF NATURAL PRODUCTS, vol. 62, no. 7, 1999, pages 976-983, XP002325162 ISSN: 0163-3864
- TAKEUCHI Y ET AL: "Synthesis and antitumor activity of fused quinoline derivatives. IV. Novel 11-aminoindolo[3,2-b]quinolines" CHEMICAL AND PHARMACEUTICAL BULLETIN, PHARMACEUTICAL SOCIETY OF JAPAN, TOKYO, JP, vol. 45, no. 2, February 1997 (1997-02), pages 406-411, XP002081505 ISSN: 0009-2363
- CHEN J ET AL: "Synthesis and cytotoxic activity of N-(2- Diethylamino)ethylcarboxam ide and other derivatives of 10H-Quindoline" BIOORGANIC & MEDICINAL CHEMISTRY, ELSEVIER SCIENCE LTD, GB, vol. 10, no. 7, 2002, pages 2381-2386, XP002325164 ISSN: 0968-0896

## Description

This invention relates to new compounds comprising one or two halogenated quindoline moieties. Such compounds can be used for the treatment of cancer.

### BACKGROUND OF THE INVENTION

Intercalators are planar polycyclic molecules and have been used as anticancer drugs. Many such molecules are known in the art. Indolo[3,2-b]quinoline is an aromatic four-membered ring system which intercalates into DNA base pairs. Many derivatives with different substitutions patterns have been synthesized and tested for biological activity against cancer (Guyen et al., Org. Biomol. Chem. 2004, 2, 981-988; Onyeibor et al., J. Med. Chem. 2005, 48, 2701-2709). Quindoline backbone numeration is the following one: 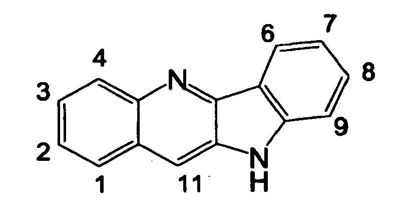 Aymami et al. (Spanish patent application P200302821;
PCT/EP2004/013106) disclose intercalating compounds with a general formula

G₁-L-G₂ (I)

wherein, comprised in a larger chemical space, G₁ and G₂ can be polycyclic moieties of the general formula 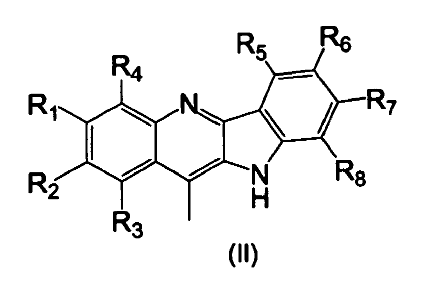 wherein -R₁ to -R₁₂ represent radicals, same or different, selected from the group consisting of H, (C₁-C₄)-alkyl, (C₁-C₄)-alkoxy, (C₁-C₄)-alkylamino, phenyl, F, Cl, Br, amino, hydroxy, and nitro.

Yang et al. (J. Nat. Prod. 1999, 62, 976-983) disclose monomeric quindoline derivatives with a bromine substitution in the 2 position, in an analysis of the cytotoxic potential of such compounds. The authors of said publication find a decreased activity of the 2-brominated compounds.

Cheng et al. (J. Chem. Soc. Perkin Trans. 1998, 1, 1619-1625) report the synthesis of halogenated cryptoleptine derivatives in a study of reaction mechanisms without any further investigation into their pharmaceutical properties.

Caprio et al. (Bioorg & Med. Chem. Lett. 2000, 10, 2063-2066) disclose a 2-brominated quindoline monomer as an intermediate.

Wright et al. (J. Med. Chem. 2001, 44, 3187-3194) report a 2-brominated cryptolepine monomer with apparently lower (compared to unsubstituted analogues) activity in an antimalarial assay.

Apart from the quindoline and cryptolepine (N5-methyl-quindoline) dimers disclosed in Aymami et al. (see reference above), dimers of quindolines are known in the state of the art; among them the natural compound cryptomisrine (Sharaf et al., J. Heterocyclic Chem. 1996, 33, 789-797).

Efforts are being made to find novel therapies that target mechanisms of oncogenesis and malignancy; however many cancers are still treated mainly with cytotoxic or anti proliferative agents such as intercalators or other drugs, which bind to or interfere with DNA. Despite the large number of potential cytotoxic drugs available as candidates for anticancer drugs, only a small subset of these are used in the clinic due to their side effects and general toxicity.

A general quality of a candidate compound is the comparison of its cytotoxicity in malignant cells versus non-malignant cells. This ratio is also referred to as the therapeutic window. Finding novel and effective compounds as cytotoxic agents with an advantageous therapeutic window is thus a highly desirable undertaking.

It is the objective of the present invention to provide effective antitumour agents with an advantageous therapeutic window.

### DESCRIPTION OF THE INVENTION

In a previous study, from a large number of compounds tested, the bis-quindoline compound **Ia** showed a modest cytotoxic activity: IC₅₀(Jurkat) = 0.97µM; IC₅₀(GLC-4) = 1.45µM; IC₅₀(NIH-3T3) = 6.77µM. IC₅₀ is the compound concentration at which 50% of a specific cell type die in a standardized assay, and thus a measure of cytotoxicity. Jurkat is a human leukaemia T cell line, GLC-4 is a human small-cell lung-carcinoma cell line and NIH-3T3 is a rat non-cancerous cell line.

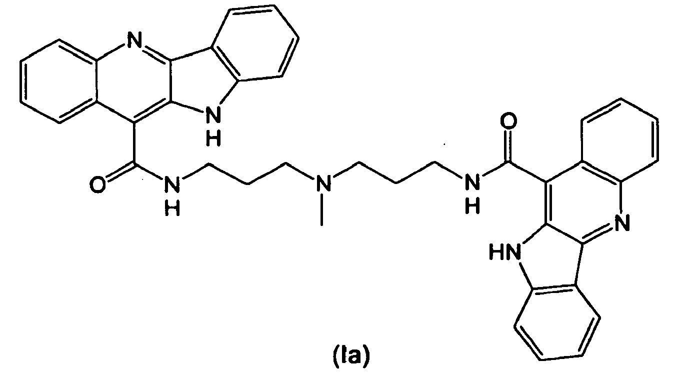

We had synthesized various novel or previously described 2-halogenated indolo[3,2-b]quinoline derivatives **(IIa-c)** and tested their activity in cell viability in two cancerous (Jurkat and GLC4) and one non-cancerous (NIH-3T3) cell lines. The compounds showed moderate activity, with all IC₅₀ higher than 10 µM (table 1).

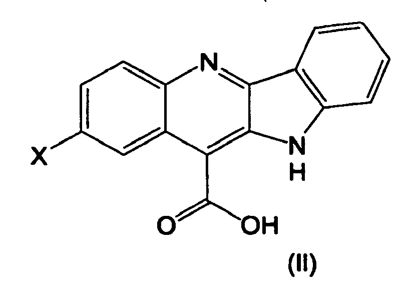

**IIa** X=Cl

**IIb** X=F

**IIc** X=Br

**Table 1. Biological activities of IIa-c.**

| **Compound** | **IC₅₀ (**µ**M)Jurkat** | **IC₅₀ (**µ**M)GLC-4** | **IC₅₀ (µM) NIH-3T3** |
|---|---|---|---|
| I**Ia** | > 25 | 15.72 | n.d. |
| **IIb** | 13.02 | 12.04 | n.d. |
| **IIc** | >25 | n.d. | n.d. |

In an attempt to elucidate the relationship between structural composition and activity of a number of quindoline derivatives, we have synthesized and evaluated dimers of 2-halogenated indolo[3,2-b]quinoline derivatives **(III-e)** and dimers containing one 2-halogenated and one non-halogenated indolo[3,2-b]quinoline derivative. To our surprise, we found that these compounds, particularly the dihalogenated dimers, show greatly enhanced activity when compared with their monomeric counterparts (table 2), but also show an enlarged therapeutic window with comparison to compound **Ia** (see above). Particularly, compound **IIIa** combines both great inhibitory activity and a large therapeutic window.

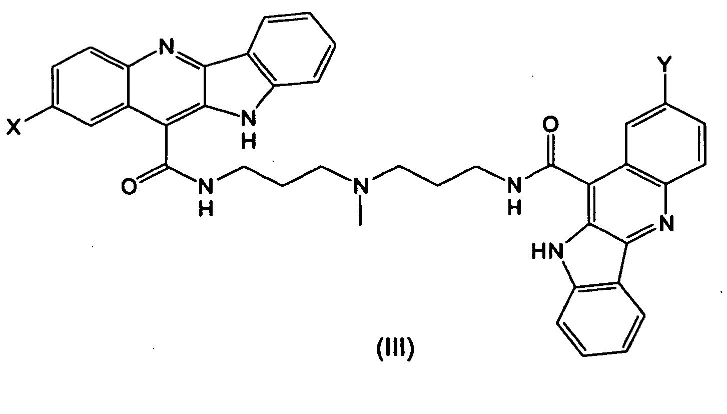

**IIIa** X=Y= Cl

**IIIb** X=Y= F

**IIIc** X=Y= Br

**IIId** X= Br; Y= Cl

**IIIe** X= F; Y= Cl

**IIIf** X= F; Y= H

**IIIg** X= Cl; Y= H

These compounds have been tested in a greater number of different cell lines. IC₅₀ is the compound concentration at which 50% of a specific cell type die in a standardized assay, and thus a measure of cytotoxicity. Jurkat is a human leukaemia T cell line, GLC-4 is a human small-cell lung-carcinoma cell line, NIH-3T3 is a rat non-cancerous cell line, K-562 is a human chronic myelogenous leukemia cell line, A549 is a human lung carcinoma cell line, RPMI-1788 is a human leukocytes of peripherial blood from healthy male cell line, MCF-7 is a human breast adenocarcinoma cell line, Capan-2 is a human pancreas adenocarcinoma cell line, Colo320HSR is a human colon carcinoma cell line, SW-837 is a human rectum adenocarcinoma cell line, IM-9 is a human bone marrow myeloma cell line, IMR-32 is a human neuroblastoma cell line and MT-4 is a human mixed heart's and peripheral blood's lymphocytes of patients with T-cell leukemia cell line.

**Table 2. Biological activities of IIIa-IIIg.**

| | **IC50 (**µ**M)** | | | | | | |
|---|---|---|---|---|---|---|---|
| | **IIIa** | **IIIb** | **IIIc** | **IIId** | **IIIe** | **IIIf** | **IIIg** |
| **Jurkat** | 0.09 | 0.5 | 0.8 | 0.6 | 0.5 | 0.2 | 0.7 |
| **GLC-4** | 0.16 | n.d. | n.d. | n.d. | n.d. | n.d. | n.d. |
| **NIH-3T3** | 11.22 | 64.29 | n.d. | n.d. | n.d. | n.d. | n.d. |
| **K-562** | 0.65 | 1.1 | n.d. | 5.3 | 6.1 | 2.8 | 5.8 |
| **A549** | 12.2 | 10.0 | n.d. | 12.9 | 11.6 | 1.6 | 6.2 |
| **RPMI-1788** | 3.0 | 5.3 | 9.1 | 3.5 | 2.9 | 1.7 | 3.2 |
| **MCF-7** | 3.4 | 4.8 | n.d. | n.d. | n.d. | n.d. | n.d. |
| **Capan-2** | 20.1 | 25.5 | n.d. | n.d. | n.d. | n.d. | n.d. |
| **Colo-320HSR** | 0.45 | 3.2 | n.d. | n.d. | n.d. | n.d. | n.d. |
| **SW-837** | 6.2 | 17.9 | n.d. | n.d. | n.d. | n.d. | n.d. |
| **IM-9** | 4.6 | 7.5 | n.d. | n.d. | n.d. | n.d. | n.d. |
| **IMR-32** | 0.9 | 0.7 | n.d. | n.d. | n.d. | n.d. | n.d. |
| **MT-4** | 4.5 | 9.0 | n.d. | n.d. | n.d. | n.d. | n.d. |

Much to the surprise of the inventors of the present invention it was found that by linking two 2-halogenated indolo[3,2-b]quinoline moieties through a linker, or one 2-halogenated indolo[3,2-b]quinoline moiety with one non-halogenated indolo[3,2-b]quinoline moiety, the resulting compound is more active in the relevant assays when compared to the monomers.

### DESCRIPTION OF THE FIGURES:

FIG. 1 is a flow chart describing a general preparation of quindoline dimers of general formula Va.
FIG. 2 is a flow chart describing a general preparation of quindoline dimers of general formula Vb.

### SUMMARY OF THE INVENTION

Halogenated quindoline dimers of the general formula (IV) are described: 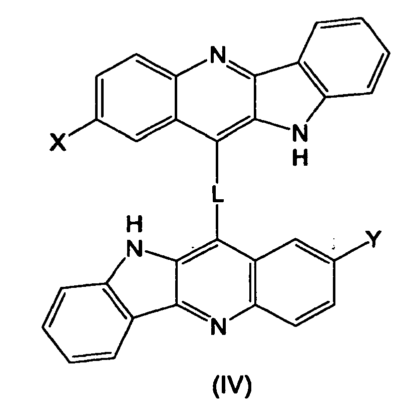 or a pharmaceutically acceptable salt thereof, wherein
-X and -Y represent atoms same or different, selected from the group consisting of -F, -Cl -Br, and -H, with the proviso that when X is -H, then Y is different from -H; and
-L- is a linker that binds covalently the two quindoline moieties, which may be branched or unbranched, which may contain one or more ether, amino or amido groups and which may be optionally substituted with one or more alcohol, alkoxy or amino groups, substituted or unsubstituted.

Halogenated quindoline dimers of the general formula (V) are described: 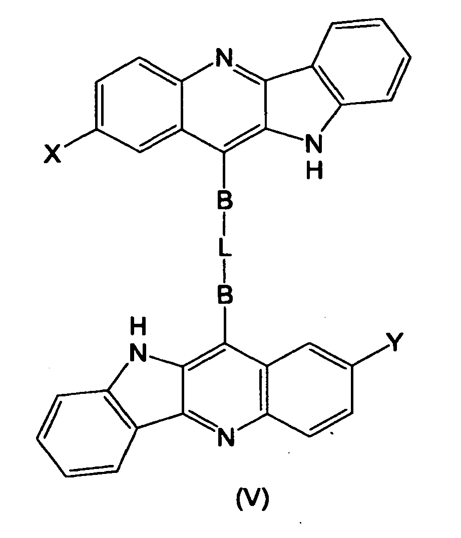 or a pharmaceutically acceptable salt thereof, wherein
-X and -Y represent atoms same or different, selected from the group consisting of -F, -Cl and -Br, and -H, with the proviso that when X is -H, then Y is different from -H;
-B- is a moiety selected from the group consisting of -CONH- and -NR'-, wherein -R' is selected from the group consisting of H, (C₁-C₄)-alkyl, (C₁-C₄)-alkoxy and (C₁-C₄)alkylamino; and
-L- is a linker selected from the following ones:
   -(CH₂)ᵣNR"(CH₂)ₛ-
   -(CH₂)ᵣNR"(CH₂)ₛNR"'(CH₂)ₜ-
-R" and -R'" are chemical groups, same or different, selected from the group consisting of H and (C₁-C₃)-alkyl; r is an integer from 1 to 3; s is an integer from 1 to 3; t is an integer from 1 to 3.

In a preferred embodiment of the invention, a halogenated quindoline dimer is provided of the general formula: 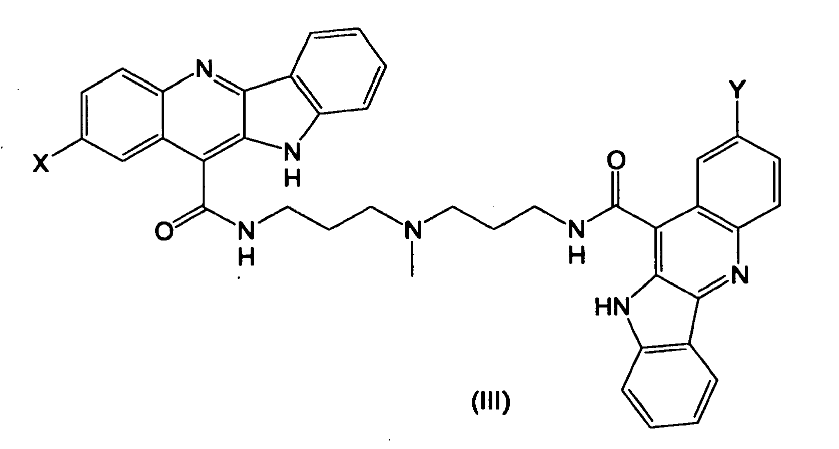 or a pharmaceutically acceptable salt thereof, wherein
-X and -Y represent atoms same or different, selected from the group consisting of -F, -Cl -Br, and -H, with the proviso that when X is -H, then Y is different from -H.

Preferably -X and -Y represent atoms same or different, selected from the group consisting of -F, -Cl and -Br.

In a second aspect, the invention provides a pharmaceutical composition comprising, as active ingredient, a therapeutically effective amount of the compound of formula III-V together with suitable pharmaceutical excipients and carriers.

A further aspect of the present invention provides a method of treatment of any type of cancer, comprising the step of administering an anti-tumor effective dose of a compound of the present invention to a mammal in need of such treatment. The compound of the invention may be administered simultaneously or sequentially with one or more other anti-neoplastic agents.

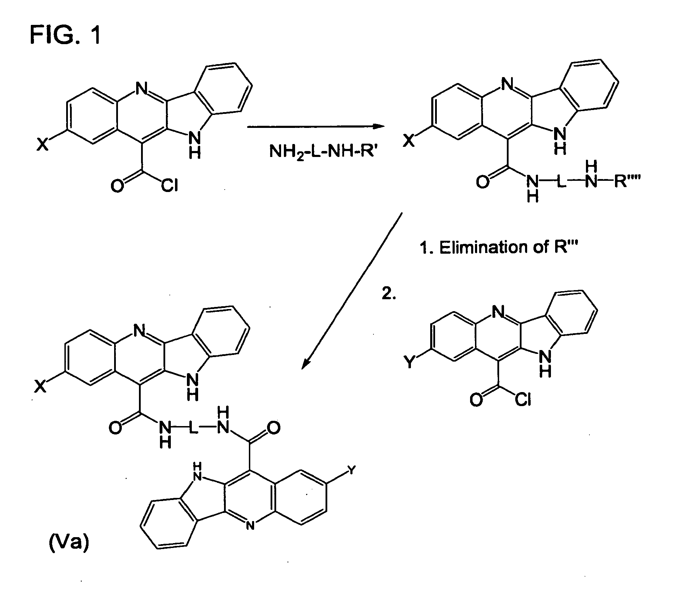

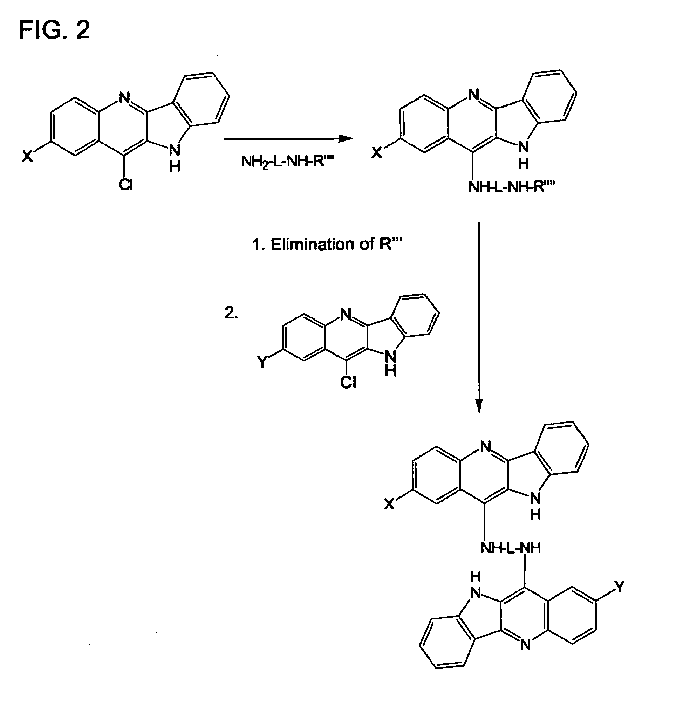

Compounds of formula V where
-B- is -CONH- (Va) can be prepared according to the process summarized in FIG.1.
-B- is -NR'- (Vb) can be prepared according to the process summarized in FIG.2.

Compounds of formula (V) may be converted into pharmaceutically acceptable acid addition salts, and salts may be converted into free compounds, by conventional methods. For instance, the acid addition salts may be prepared by contacting the free base with an appropriate amount of the desired acid in a conventional manner.

Compounds of formula (III) are, as illustrated by biological results in example 8, active for cancer treatment.

In another aspect, the present invention provides the use of a compound of formula (III) as defined above for the manufacture of a medicament for the treatment of cancer in a patient needed thereof.

Preferably, the cancer is selected from Jurkat leukemia, chronic myelogenous leukemia, lung carcinoma, breast adenocarcinoma, pancreas adenocarcinoma, colon carcinoma, rectum adenocarcinoma, bone marrow myeloma or a neuroblastoma.

### EXAMPLES

### Example 1: Preparation of N,N'-(4-methyl-4-azaheptamethylene)-di-(-2-chloro-10H-indolo[3,2-b]quinoline)-11,11'-arboxamide (IIIa)

2-Chloro-10H-indolo[3,2-b]quinoline-11-carboxylic acid previously prepared (0.5 g, 1.8 mmol) (Ablordeppey et al., Bioorg. Med. Chem. 2002, 5, 1337-1346) and was dissolved in 20 ml of refluxing SOCl₂. After 6 hours a clear solution was obtained, and all volatiles were removed in vacuo. The crude acid chloride was suspended in 20 ml of CH₂Cl₂. A solution of [3-[(3-aminopropyl)methylamino]propyl]carbamic acid tert-butyl ester previously prepared (0.6 g, 2.4 mmol) (Spicer at el., Bioorg. Med. Chem. 2002, 10, 19-29) and triethylamine (1.2 ml) in 20 ml of CH₂Cl₂ was added at room temperature, and the mixture was stirred for 14 hours. The organic phase was washed (NaHCO₃, brine) and dried (MgSO₄). Removal of the solvent and purification by chromatography on alumina (CH₂Cl₂/MeOH, 90:10) yielded [2-[[2-[(2-chloro-10H-Indolo[3,2-b]quinoline-11-carbonyl)amino]ethyl]methylamino)ethyl]carbamic acid tert-butyl ester (0.7 g, 71%) as a viscous oil. ¹H-NMR [MeOD, δ, ppm]: 8.45 (d, J = 7.8 Hz, 1H), 8.22 (m, 2H ), 7.68 (m, 4H), 7.35 (dd, J = 8.0 Hz, 1H), 3.70 (t, J = 6.9 Hz, 2H, CH₂NO), 3.05 (t, J = 6.9 Hz, 2H, CH₂NHBOC), 2.65 (m, 2H, CH₂N(CH₃)), 2.50 (m, 2H, CH₂N(CH₃)), 2.33 (s, 3H, N(CH₃)), 1.69 (m, 4H, 2CH₂CN(CH₃)), 1.40 (s, 9H, C(CH₃)₃).

To a solution of [2-[[2-[(2-chloro-10H-indolo[3,2-b]quinoline-11-carbonyl)amino]-ethyl]methylamino)ethyl]-carbamic acid tert-butyl ester (0.7 g, 1.3 mmol) in CH₂Cl₂ (50 ml), was added trifluoroacetic acid (16 ml). The reaction mixture was stirred at room temperature for 16 h at which point the reaction was completed by TLC. All solvents were removed under reduced pressure to give 2-chloro-10H-indolo[3,2-b]quinoline-[3-[(3-aminopropyl)methylamino]propyl]-11-carboxamide (0.54 g , 96%) as an oil, which was used directly in the next reaction. ¹H-NMR [MeOD, δ, ppm]: 8.46 (d, J = 7.8 Hz, 1H), 8.28 (m, 2H), 7.90 (m, 1H ), 7.82 (m, 2H), 7.66 (d, J = 8.0 Hz, 1H), 7.44 (m, 1H), 3.79 (t, J = 6.9 Hz, 2H, CH₂NO), 3.09 (m, 2H, CH₂NH₂), 2.98 (s, 3H, N(CH₃)), 2.28 (m, 4H, CH₂N(CH₃)), 2.16 (m, 4H, 2CH₂CN(CH₃)). 2-Chloro-10H-indolo[3,2-b]quinoline-11-carboxylic acid previously prepared (0.3 g, 0.97 mmol) (Ablordeppey et al., see above), and was dissolved in 14 ml of refluxing SOCl₂. After 6 hours a clear solution was obtained, and all volatiles were removed in vacuo. The crude acid chloride was suspended in 15 ml of CH₂Cl₂. A solution of 2-Chloro-10H-indolo[3,2-b]quinoline-[3-[(3-aminopropyl)methyl-amino]propyl]-11-carboxamide (0.54 g, 1.27 mmol) and triethylamine (1.5 ml) in 15 ml of CH₂Cl₂ was added at room temperature, and the reaction mixture was stirred for 14 hours. The organic phase was washed (NaHCO₃, brine) and dried (MgSO₄). Removal of the solvent and purification by chromatography on alumina (CH₂Cl₂/MeOH, 95:5) yielded the desired compound (0.12 g, 13%) as a solid. mp >300°C. ¹H-NMR [CDCl₃, δ, ppm]: 8.36 (m, 1H), 8.09 (m, 7H), 7.59 (m, 7H ), 7.28 (m, 1H), 3.67 (m, 4H, 2CH₂NO), 2.58 (m, 4H, 2CH₂NO), 2.30 (s, 3H, N(CH₃)), 1.96 (m, 4H, 2CH₂CN(CH₃)); MS (EI, m/z) 703 (M⁺+1).

### Example 2: Preparation of N,N'-(4-methyl-4-azaheptamethylene)-di-(-2-fluoro-10H-indolo[3,2-b]quinoline)-11,11'-carboxamide (IIIb)

2-Fiuoro-10H-indoio[3,2-b]quinoline-11-carboxylic acid previously prepared (0.5 g, 1.8 mmol) (Ablordeppey et al., see above), and was dissolved in 20 ml of refluxing SOCl₂. After 6 hours a clear solution was obtained, and all volatiles were removed in vacuo. The crude acid chloride was suspended in 20 ml of CH₂Cl₂. A solution of [3-[(3-aminopropyl)methylamino]propyl]carbamic acid tert-butyl ester previously prepared (0.6 g, 2.4 mmol) (Spicer at el., see above) and triethylamine (1.2 ml) in 20 ml of CH₂Cl₂ was added at room temperature, and the mixture was stirred for 14 hours. The organic phase was washed (NaHCO₃, brine) and dried (MgSO₄). Removal of the solvent and purification by chromatography on alumina (CH₂Cl₂MeOH, 90:10) yielded [2-[[2-[(2-fluoro-10H-Indolo[3,2-b]quinoline-11-carbonyl)amino]ethyl]methylamino)ethyl]carbamic acid tert-butyl ester (0.6 g, 71 %) as a viscous oil. ¹H-NMR [MeOD, δ, ppm]: 8.34 (d, J = 7.8 Hz, 1H), 8.14 (m, 2H ), 7.74 (m, 4H), 7.28 (dd, J = 8.0 Hz, 1H), 3.66 (t, J = 6.9 Hz, 2H, CH₂NO), 3.05 (t, J = 6.9 Hz, 2H, CH₂NHBOC), 2.59 (m, 2H, CH₂N(CH₃)), 2.48 (m, 2H, CH₂N(CH₃)), 2.31 (s, 3H, N(CH₃)), 1.67 (m, 4H, 2CH₂CN(CH₃)), 1.40 (s, 9H, C(CH₃)₃).

To a solution of [2-[[2-[(2-fluonr10H-indolo[3,2-b]quinoline-11-carbonyl)amino]-ethyl]methylamino)ethyl]-carbamic acid tert-butyl ester (0.7 g, 1.3 mmol) in CH₂Cl₂ (50 ml), was added trifluoroacetic acid (16 ml). The reaction mixture was stirred at room temperature for 16 h at which point the reaction was completed by TLC. All solvents were removed under reduced pressure to give 2-fluoro-10H-indolo[3,2-b]quinoline-[3-[(3-aminopropyl)methylamino]propyl]-11-carboxamide (0.5 g , 99%) as an oil, which was used directly in the next reaction. ¹H-NMR [MeOD, δ, ppm]: 8.41 (d, J = 7.8 Hz, 1H), 8.23 (m, 2H), 7.96 (m, 1H ), 7.74 (m, 2H), 7.71 (d, J = 8.0 Hz, 1H), 7.49 (m, 1H), 3.77 (t, J = 6.9 Hz, 2H, CH₂NO), 3.06 (m, 2H, CH₂NH₂), 2.98 (s, 3H, N(CH₃)), 2.28 (m, 4H, CH₂N(CH₃)), 2.18 (m, 4H, 2CH₂CN(CH₃)). 2-Fluoro-10H-indolo[3,2-b]quinoline-11-carboxylic acid previously prepared (0.3 g, 0.93 mmol) (Ablordeppey et al., see above), and was dissolved in 14 ml of refluxing SOCl₂. After 6 hours a clear solution was obtained, and all volatiles were removed in vacuo. The crude acid chloride was suspended in 15 ml of CH₂Cl₂. A solution of 2-Fluoro-10H-indolo[3,2-b]quinoline-[3-[(3-aminopropyl)methyl-amino]propyl]-11-carboxamide (0.5 g, 1.2 mmol) and triethylamine (1.5 ml) in 15 ml of CH₂Cl₂ was added at room temperature, and the reaction mixture was stirred for 14 hours. The organic phase was washed (NaHCO₃, brine) and dried (MgSO₄). Removal of the solvent and purification by chromatography on alumina (CH₂Cl₂/MeOH, 95:5) yielded the desired compound (0.13 g, 16%) as a solid. mp 170 -172°C. ¹H-NMR [CDCl₃, δ, ppm]: 8.36 (m, 1H), 8.23 (m, 7H), 8.01 (m, 7H ), 7.63 (m, 1H), 3.69 (m, 4H, 2CH₂NO), 2.81 (m, 4H, 2CH₂NO), 2.37 (s, 3H, N(CH₃)), 2.06 (m, 4H, 2CH₂CN(CH₃)); MS (El, m/z) 670 (M⁺+1).

### Example 3: Preparation of N,N'-(4-methyl-4-azaheptamethylene)-di-(-2-bromo-10H-indolo[3,2-b]quinoline)-11,11'-carboxamide (IIIc)

2-Bmmo-10H-indolo[3,2-b]quinoline-11-carboxylic acid previously prepared (0.5 g, 1.4 mmol) (Caprio et al., see above), and was dissolved in 20 ml of refluxing SOCl₂. After 6 hours a clear solution was obtained, and all volatiles were removed in vacuo. The crude acid chloride was suspended in 20 ml of CH₂Cl₂. A solution of [3-[(3-aminopropyl)methylamino]propyl]carbamic acid tert-butyl ester previously prepared (0.4 g, 1.8 mmol) (Spicer at el., see above) and triethylamine (1.2 ml) in 20 ml of CH₂Cl₂ was added at room temperature, and the mixture was stirred for 14 hours. The organic phase was washed (NaHCO₃, brine) and dried (MgSO₄). Removal of the solvent and purification by chromatography on alumina (CH₂Cl₂/MeOH, 90:10) yielded [2-[[2-[(2-bromo-10H-Indolo[3,2-b]quinoline-11-carbonyl)amino]ethyl]methylamino)ethyl]carbamic acid tert-butyl ester (0.5 g, 66%) as a viscous oil . ¹H-NMR [MeOD, δ, ppm]: 8.47 (d, J = 7.8 Hz, 1H), 8.15 (m, 2H ), 7.81 (m, 4H), 7.36 (dd, J = 8.0 Hz, 1H), 3.71 (t, J = 6.9 Hz, 2H, CH₂NO), 3.05 (t, J = 6.9 Hz, 2H, CH₂NHBOC), 2.52 (m, 2H, CH₂N(CH₃)), 2.51 (m, 2H, CH₂N(CH₃)), 2.35 (s, 3H, N(CH₃)), 1.71 (m, 4H, 2CH₂CN(CH₃)), 1.40 (s, 9H, C(CH₃)₃)

To a solution of [2-[[2-[(2-bromo-10H-indolo[3,2-b]quinoline-11-carbonyl)amino]-ethyl]methylamino)ethyl]-carbamic acid tert-butyl ester (0.5 g, 0.9 mmol) in CH₂Cl₂ (50 ml), was added trifluoroacetic acid (16 ml). The reaction mixture was stirred at room temperature for 16 h at which point the reaction was completed by TLC. All solvents were removed under reduced pressure to give 2-bromo-10H-indolo[3,2-b]quinoline-[3-[(3-aminopropyl)methylamino]propyl]-11-carboxamide (0.4 g , 97%) as an oil, which was used directly in the next reaction. ¹H-NMR [MeOD, δ, ppm]: 8.42 (d, J = 7.8 Hz, 1H), 8.16 (m, 2H), 7.97 (m, 1H), 7.80 (m, 2H), 7.64 (d, J = 8.0 Hz, 1H), 7.40 (m, 1H), 3.78 (t, J = 6.9 Hz, 2H, CH₂NO), 3.06 (m, 2H, CH₂NH₂), 2.92 (s, 3H, N(CH₃)), 2.28 (m, 4H, CH₂N(CH₃)), 2.18 (m, 4H, 2CH₂CN(CH₃)). 2-Bromo-10H-indolo[3,2-b]quinoline-11-carboxylic acid previously prepared (0.24 g, 0.73 mmol) (Caprio et al., see above), and was dissolved in 14 ml of refluxing SOCl₂. After 6 hours a clear solution was obtained, and all volatiles were removed in vacuo. The crude acid chloride was suspended in 15 ml of CH₂Cl₂. A solution of 2-Bromo-10H-indolo[3,2-b]quinoline-[3-[(3-aminopropyl)methyl-amino]propyl]-11-carboxamide (0.4 g, 0.94 mmol) and triethylamine (1.5 ml) in 15 ml of CH₂Cl₂ was added at room temperature, and the reaction mixture was stirred for 14 hours. The organic phase was washed (NaHCO₃, brine) and dried (MgSO₄). Removal of the solvent and purification by chromatography on alumina (CH₂Cl₂/MeOH, 95:5) yielded the desired compound (0.12 g, 16%) as a solid. mp 110 -112°C. ¹H-NMR [CDCl₃, δ, ppm]: 8.25 (m, 1H), 8.15 (m, 7H), 8.03 (m, 7H), 7.35 (m, 1H), 3.71 (m, 4H, 2CH₂NO), 2.81 (m, 4H, 2CH₂NO), 2.48 (s, 3H, N(CH₃)), 2.06 (m, 4H, 2CH₂CN(CH₃)); MS (El, m/z) 792 (M⁺+1).

### Example 4: Preparation of N-[3-[[3-[11-((2-bromo-10H-indolo[3,2-b]quinoline)carbonyl)amino]-propyl]-methylamino]propyl]-2-chloro-10H-indolo[3,2-b]quinoline-11-carboxamide (IIId)

2-Bromo-10H-indolo[3,2-b]quinoline-11-carboxylic acid previously prepared (39 mg, 0.23 mmol) (see example 3) was dissolved in 15 ml of refluxing SOCl₂. After 6 hours, a clear solution was obtained and all volatiles were removed in vacuo. The crude acid chloride was suspended in 15 ml of CH₂Cl₂. A solution of 2-Chloro-10H-indolo[3,2-b]quinoline-[3-[(3-aminopropyl)methylamino]propyl]-11-carboxamide previously prepared (0.13 mg, 0.3 mmol) (see example 1) and triethylamine (1.5 ml) in 15 ml of CH₂Cl₂ was added at room temperature and the reaction mixture was stirred for 14 hours. The organic phase was washed (NaHCO₃, brine) and dried (MgSO₄). Removal of the solvent and purification by chromatography on alumina (CH₂Cl₂/MeOH, 95:5) yielded the desired compound (0.16 g, 94%) as a yellow solid. m. p. 110-115°C. ¹H-NMR [CDCl_{3,} δ, ppm]: 8.42 (m, 2H), 8.24 (m, 4H), 7.69-7.30 (m, 8H), 7.20 (m, 2H) 3.74 (m, 4H, 2CH₂NO), 2.73-2.62 (m, 4H, 2CH₂NH), 2.43 (s, 3H, CH₃), 2.04 (m, 4H, 4CH₂). MS (El, *m*/*z*) 748 (M⁺+1).

### Example 5: Preparation of N-[3-[[3-[11-((2-chloro-10H-indolo[3,2-b]quinoline)carbonyl)amino]-propyl]-methylamino]propyl]-2-fluoro-10l-l-indolo[3,2-b]quinoline-11-carboxamide (IIIe)

2-Fluoro-10H-indolo[3,2-b]quinoline-11-carboxylic acid previously prepared (69 mg, 0.25 mmol) (see example 2) was dissolved in 15 ml of refluxing SOCl₂. After 6 hours, a clear solution was obtained and all volatiles were removed in vacuo. The crude acid chloride was suspended in 15 ml of CH₂Cl₂. A solution of 2-Chloro-10H-indolo[3,2-b]quinoline-[3-[(3-aminopropyl)methylamino]propyl]-11-carboxamide previously prepared (0.14 mg, 0.33 mmol) (see example 1) and triethylamine (1.5 ml) in 15 ml of CH₂Cl₂ was added at room temperature and the reaction mixture was stirred for 14 hours. The organic phase was washed (NaHCO₃, brine) and dried (MgSO₄). Removal of the solvent and purification by chromatography on alumina (CH₂Cl₂/MeOH, 95:5) yielded the desired compound (11 mg, 7%) as a foam. ¹H-NMR [CDCl₃, δ, ppm]: 8.46 (m, 2H), 8.23 (m, 4H), 8.01-7.36 (m, 8H ), 7.24 (m, 2H) 3.71 (m, 4H, 2CH₂NO), 2.71-2.60 (m, 4H, 2CH₂NH), 2.40 (s, 3H, CH₃), 2.06 (m, 4H, 4CH₂). MS (El, *m*/*z*) 687 (M⁺+1).

### Example 6: Preparation of N-[3-[[3-[11-((2-fluoro-10H-indolo[3,2-b]quinoline)carbonyl)amino]-propyl]-methylamino]propyl]-10H-indolo[3,2-b]quinoline-11-carboxamide (IIIf)

2-Fluoro-10H-indolo[3,2-b]quinoline-11-carboxylic acid previously prepared (37 mg, 0.13 mmol) (see example 2) was dissolved in 15 ml of refluxing SOCl₂. After 6 hours, a clear solution was obtained and all volatiles were removed in vacuo. The crude acid chloride was suspended in 15 ml of CH₂Cl₂. A solution of 10H-indolo[3,2-b]quinoline-[3-[(3-aminopropyl)methylamino]propyl]-11-carboxamide previously prepared (68 mg, 0.17 mmol) (Aymami et al. Spanish patent application P200302821; PCT/EP2004/013106) and triethylamine (1.5 ml) in 15 ml of CH₂Cl₂ was added at room temperature and the reaction mixture was stirred for 14 hours. The organic phase was washed (NaHCO₃, brine) and dried (MgSO₄). Removal of the solvent and purification by chromatography on alumina (CH₂Cl₂/MeOH, 95:5) yielded the desired compound as a yellow solid, (32 mg, 86%), mp > 200°C. ¹H-NMR [CDCl₃, δ, ppm]: 8.43 (m, 2H), 8.10 (m, 4H), 7.66-7.23 (m, 8H ), 7.22 (m, 2H) 3.67 (m, 4H, 2CH₂NO), 2.71 (m, 4H, 2CH₂NH), 2.40 (s, 3H, CH₃), 2.01 (m, 4H, 4CH₂). MS (EI, *m*/*z*) 652 (M⁺+1).

### Example 7: Preparation of N-[3-[[3-[11-((2-chloro-10H-indolo[3,2-b]quinoline)carbonyl)amino]-propyl]-methylamino]propyl]-10H-indolo[3,2-b]quinoline-11-carboxamide (IIIg)

2-Chloro-10H-indolo[3,2-b]quinoline-11-carboxylic acid previously prepared (0.23 g, 0.76 mmol) (see example 1) was dissolved in 15 ml of refluxing SOCl₂. After 6 hours, a clear solution was obtained and all volatiles were removed in vacuo. The crude acid chloride was suspended in 15 ml of CH₂Cl₂. A solution of 10H-indolo[3,2-b]quinoline-[3-[(3-aminopropyl)methylamino]propyl]-11-carboxamide previously prepared (0.39 g, 1 mmol) (Aymami et al. Spanish patent application P200302821; PCT/EP2004/013106) and triethylamine (1.5 ml) in 15 ml of CH₂Cl₂ was added at room temperature and the reaction mixture was stirred for 14 hours. The organic phase was washed (NaHCO₃, brine) and dried (MgSO₄). Removal of the solvent and purification by chromatography on alumina (CH₂Cl₂/MeOH, 95:5) yielded the desired compound as a yellow solid, (0.3 g, 56%, m. p. 105-110°C. ¹H-NMR [CDCl₃, δ, ppm]: 8.45 (m, 2H), 8.14 (m, 4H), 7.69-7.30 (m, 8H ), 7.22 (m, 2H) 3.67 (m, 4H, 2CH₂NO), 2.74-2.63 (m, 4H, 2CH₂NH), 2.34 (s, 3H, CH₃), 2.03 (m, 4H, 4CH₂). MS (EI, *m*/*z*) 669 (M⁺+1).

### Example 8: In vitro biological test

The in vitro cytotoxicity of the compounds of the present invention was evaluated by colorimetric assays with tetrazol salts (MTT) on Jurkat clon E6-1 leukemia, on GLC-4 clon carcinoma, on K-562 chronic myelogenous leukemia, on A549 lung carcinoma, on RPMI-1788 leukocytes of peripherial blood from healthy male, on MCF-7 breast adenocarcinoma, on Capan-2 pancreas adenocarcinoma, on Colo320HSR colon carcinoma, on SW-837 rectum adenocarcinoma, on IM-9 bone marrow myeloma, on IMR-32 neuroblastoma and on MT-4 mixed heart's and peripheral blood's lymphocytes of patients with T-cell leukemia human cell lines, according to the protocol described by Mosmann et al. (J. Immunol. Methods, 1983,65, 55-63) incorporated herein by reference. In all cases the concentrations used were until 100 µM and times of incubation until 72 h. Table 3 shows the biological activity (IC₅₀) of these compounds.

**Table 3. Biological activities.**

| | **IC50 (**µ**M)** | | | | | | |
|---|---|---|---|---|---|---|---|
| | **IIIa** | **IIIb** | **IIIc** | **IIId** | **IIIe** | **IIIf** | **IIIg** |
| **Jurkat** | 0.09 | 0.5 | 0.8 | 0.6 | 0.5 | 0.2 | 0.7 |
| **GLC-4** | 0.16 | n.d. | n.d. | n.d. | n.d. | n.d. | n.d. |
| **NIH-3T3** | 11.22 | 64.29 | n.d. | n.d. | n.d. | n.d. | n.d. |
| **K-562** | 0.65 | 1.1 | n.d. | 5.3 | 6.1 | 2.8 | 5.8 |
| **A549** | 12.2 | 10.0 | n.d. | 12.9 | 11.6 | 1.6 | 6.2 |
| **RPMI-1788** | 3.0 | 5.3 | 9.1 | 3.5 | 2.9 | 1.7 | 3.2 |
| **MCF-7** | 3.4 | 4.8 | n.d. | n.d. | n.d. | n.d. | n.d. |
| **Capan-2** | 20.1 | 25.5 | n.d. | n.d. | n.d. | n.d. | n.d. |
| **Colo-320HSR** | 0.45 | 3.2 | n.d. | n.d. | n.d. | n.d. | n.d. |
| **SW-837** | 6.2 | 17.9 | n.d | n.d. | n.d. | n.d. | n.d. |
| **IM-9** | 4.6 | 7.5 | n.d. | n.d. | n.d. | n.d. | n.d. |
| **IMR-32** | 0.9 | 0.7 | n.d. | n.d. | n.d. | n.d. | n.d. |
| **MT-4** | 4.5 | 9.0 | n.d. | n.d. | n.d. | n.d. | n.d. |

## Claims

1. A compound of formule (III) 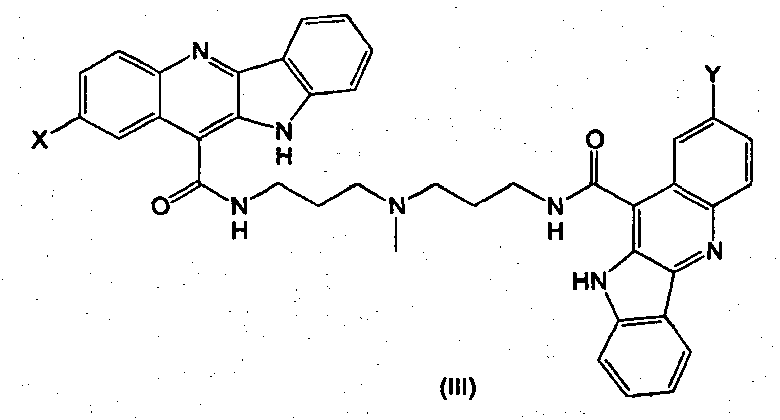 or a pharmaceutically acceptable salt thereof, wherein:
-X and -Y represent atoms same or different, selected from the group consisting of -F, -Cl, -Br, and -H, with the proviso that when X is -H, then Y is different from -H.

2. The compound of formula (III) according to claim 1 wherein -X and -Y represent atoms same or different, selected from the group consisting of -F,-Cl and -Br.

3. The compound of formula (III) according to claim 1 which is selected from the group consisting of:
N-[3-[[3-[11-((2-bromo-10H-indolo[3,2-b]quinoline)carbonyl)amino]-propyl]-methylamino]propyl]-2-chloro-10H-indolo[3,2-b]quinaline-11-carboxamide;
N-[3-[[3-[11-((2-chloro-10H-indolo[3,2-b]quinoline)carbonyl)amino]-propyl]-methylamino]propyl]-2-fluoro-10H-indolo[3,2-b]quinoline-11-carboxamide;
N-(3-[[3-[11-((2-fluoro-10H-indolo[3,2-b]quinoline)carbonyl)amino]-propyl]-methylamino]propyl]-10H-indolo[3,2-b]quinoline-11-carboxamide; and
N-[3-[[3-[11-((2-chloro-10H-indolo[3,2-b]quinoline)carbonyl)amino]-propyl]-methylamino]propyl]-10H-indolo[3,2-b]quinoline-11-carboxamide.

4. The compound of formula (III) according to claim 2 which is selected from the group consisting of:
N,N'-(4-methyl-4-azaheptamethylene)-di-(-2-chloro-10H-indolo[3,2-b]quinoline)-11,11'-carboxamide;
N,N'-(4-methyl-4-azaheptamethylene)-di-(-2-fluoro-10H-indolo[3,2-b]quinoline)-11,11'-carboxamide; and
N,N'-(4-methyl-4-azaheptamethylene)-di-(-2-bromo-10H-indolo[3,2-b]quinoline)-11,11'-carboxamide.

5. A pharmaceutical composition comprising a therapeutically effective amount of the compound as defined in any of claims 1-4, together with appropiate amounts of pharmaceutical excipients or carriers.

6. A compound of formula (III) according to any of claims 1-4 for use as a medicament.

7. Use of a compound of formula (III) as defined in any of claims 1-4 for the manufacture of a medicament for the treatment of cancer.

8. Use according to claim 7 wherein the cancer is selected from the group consisting of: leukemia, lung carcinoma, breast adenocarcinoma, pancreas adenocarcinoma, colon carcinoma, rectum adenocarcinoma, bone marrow myeloma and neuroblastoma.

## Patentansprüche

1. Verbindung der Formel (III): 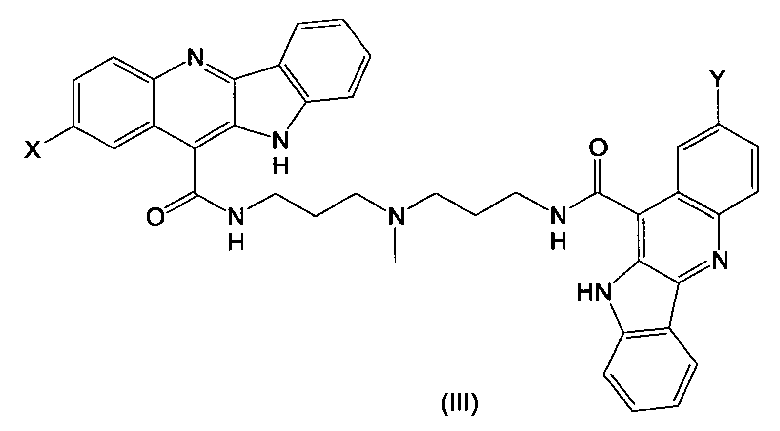 oder ein pharmazeutisch verträgliches Salz davon, worin:
-X und -Y gleichartige oder verschiedenartige Atome darstellen, die ausgewählt sind aus der Gruppe, die aus -F, -Cl, -Br, und -H besteht, unter dem Vorbehalt, dass, wenn X -H ist, Y folglich verschieden von -H ist.

2. Verbindung der Formel (III) nach Anspruch 1, worin -X und -Y gleichartige oder verschiedenartige Atome darstellen, die ausgewählt sind aus der Gruppe, die aus -F, -Cl und -Br besteht.

3. Verbindung der Formel (III) nach Anspruch 1, die aus der folgenden Gruppe ausgewählt ist:
N-[3-[[3-[11-((2-Brom-10H-indolo[3,2-b]quinolin)carbonyl)amino]propyl]-methylamino]propyl]-2-chlor-10H-indolo[3,2-b]quinolin-11-carboxamid;
N-[3-[[3-[11-((2-Chlor-10H-indolo[3,2-b]quinolin)carbonyl)amino]propyl]-methylamino]propyl]-2-fluor-10H-indolo[3,2-b]quinolin-11-carboxamid;
N-[3-[[3-[11-((2-Fluor-10H-indolo[3,2-b]quinolin)carbonyl)amino]propyl]-methylamino]propyl]-10H-indolo[3,2-b]quinolin-11-carboxamid; und
N-[3-[[3-[11-((2-Chlor-10H-indolo[3,2-b]quinolin)carbonyl)amino]propyl]-methylamino]propyl]-10H-indolo[3,2-b]quinolin-11-carboxamid.

4. Verbindung der Formel (III) nach Anspruch 2, die aus der folgenden Gruppe ausgewählt ist:
N,N'-(4-Methyl-4-azaheptamethylen)-di-(-2-chlor-10H-indolo[3,2-b]quinolin)-11,11'-carboxamid;
N,N'-(4-Methyl-4-azaheptamethylen)-di-(-2-fluor-10H-indolo[3,2-b]quinolin)-11,11'-carboxamid; und
N,N'-(4-Methyl-4-azaheptamethylen)-di-(-2-brom-10H-indolo[3,2-b]quinolin)-11,11'-carboxamid.

5. Pharmazeutische Zusammensetzung, die eine therapeutisch wirksame Menge der Verbindung, wie sie in einem der Ansprüche 1-4 definiert wird, zusammen mit angemessenen Mengen an Pharmahilfsstoffen und Arzneimittelträgern beinhaltet.

6. Verbindung der Formel (III) nach einem der Ansprüche 1-4 für die Verwendung als Medikament.

7. Verwendung einer Verbindung der Formel (III), wie in einem der Ansprüche 1-4 definiert, für die Herstellung eines Medikaments zur Krebsbehandlung.

8. Verwendung nach Anspruch 7, worin der Krebs ausgewählt ist aus der Gruppe, die aus Leukämie, Lungenkrebs, Brustdrüsenkrebs, Bauchspeicheldrüsenkrebs, Dickdarmkrebs, Adenokarzinom des Rektum, Myelom des Knochenmarks und Neuroblastom besteht.

## Revendications

1. Composé de formule (III) 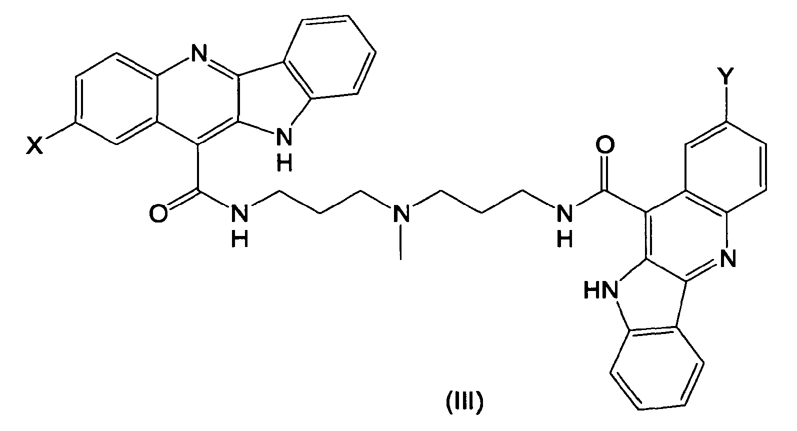 ou un sel pharmacologiquement admissible d'un tel composé, dans laquelle formule :
-X et -Y représentent des atomes identiques ou différents, choisis dans le groupe constitué de -F, -Cl, -Br et -H, à condition que lorsque X est -H, alors Y est différent de -H.

2. Composé de formule (III) selon la revendication 1, dans lequel -X et -Y représentent des atomes identiques ou différents, choisis dans le groupe constitué de -F, -Cl et -Br.

3. Composé de formule (III) selon la revendication 1, qui est choisi dans le groupe constitué de :
N-[3-[[3-[11-((2-bromo-10H-indolo[3,2-b]quinoline)carbonyl)amino]-propyl]-méthylamino]propyl]-2-chloro-10H-indolo[3,2-b]quinoline-11-carboxamide ;
N-[3-[[3-[11-((2-chloro-10H-indolo[3,2-b]quinoline)carbonyl)amino]-propyl]-méthylamino]propyl]-2-fluoro-10H-indolo[3,2-b]quinoline-11-carboxamide ;
N-[3-[[3-[11-((2-fluoro-10H-indolo[3,2-b]quinoline)carbonyl)amino]-propyl]-méthylamino]propyl]-10H-indolo[3,2-b]quinoline-11-carboxamide ; et
N-[3-[[3-[11-((2-chloro-10H-indolo[3,2-b]quinoline)carbonyl)amino]-propyl]-méthylamino]propyl]-10H-indolo[3,2-b]quinoline-11-carboxamide.

4. Composé de formule (III) selon la revendication 2, qui est choisi dans le groupe constitué de :
N,N'-(4-méthyl-4-azaheptaméthylène)-di-(-2-chloro-10H-indolo[3,2-b]quinoline)-11,11'-carboxamide ;
N,N'-(4-méthyl-4-azaheptaméthylène)-di-(-2-fluoro-10H-indolo[3,2-b]quinoline)-11,11'-carboxamide ; et
N,N'-(4-méthyl-4-azaheptaméthylène)-di-(-2-bromo-10H-indolo[3,2-b]quinoline)-11,11'-carboxamide.

5. Composition pharmaceutique comprenant une quantité efficace sur le plan thérapeutique du composé tel que défini dans l'une quelconque des revendications 1 à 4, conjointement avec des quantités appropriées d'excipients ou vecteurs pharmaceutiques.

6. Composé de formule (III) selon l'une quelconque des revendications 1 à 4 pour une utilisation en tant que médicament.

7. Utilisation d'un composé de formule (III) tel que défini dans l'une quelconque des revendications 1 à 4 pour la fabrication d'un médicament pour le traitement du cancer.

8. Utilisation selon la revendication 7, dans laquelle le cancer est choisi dans le groupe constitué de : leucémie, cancer du poumon, adénocarcinome du sein, adénocarcinome du pancréas, carcinome du côlon, adénocarcinome du rectum, myélome de moelle osseuse et neuroblastome.
